(19) Europäisches Patentamt
European Patent Office
Office européen des brevets

(11) EP 1 312 350 B1

(12) FASCICULE DE BREVET EUROPEEN

(45) Date de publication et mention de la délivrance du brevet: **07.04.2010 Bulletin 2010/14**

(51) Int Cl.:
***A61K 8/898*** *(2006.01)* ***A61Q 5/00*** *(2006.01)*
***A61Q 5/08*** *(2006.01)*

(21) Numéro de dépôt: **02292744.6**

(22) Date de dépôt: **04.11.2002**

# (54) Utilisation de silicones aminées particulières en pré- ou post-traitement de décoloration de fibres kératiniques

Verwendung von speziellen Aminosilikonen zur Vor- oder Nachbehandlung in der Entfärbung von Keratinfasern

Use or particular aminosilicones as a pre- or post-treatment in bleaching keratinous fibres

(84) Etats contractants désignés:
**AT BE BG CH CY CZ DE DK EE ES FI FR GB GR IE IT LI LU MC NL PT SE SK TR**

(30) Priorité: **08.11.2001 FR 0114468**

(43) Date de publication de la demande:
**21.05.2003 Bulletin 2003/21**

(73) Titulaire: **L'Oréal**
**75008 Paris (FR)**

(72) Inventeurs:
- **Devin-Baudoin, Priscille**
  **92170 Vanves (FR)**
- **Sabbagh, Anne**
  **92500 Rueil Malmaison (FR)**

(74) Mandataire: **Fevrier, Murielle Françoise E.**
**L'Oréal**
**D.I.P.I.**
**25-29 Quai Aulagnier**
**92600 Asnières (FR)**

(56) Documents cités:
**EP-A- 0 890 355** **DE-A- 19 754 053**
**GB-A- 2 165 550**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet l'utilisation, en pré- ou en post- traitement d'une décoloration des fibres kératiniques humaines et plus particulièrement des cheveux, d'une composition comprenant au moins une silicone aminée particulière.
Elle a aussi pour objet des procédés de décoloration des fibres kératiniques humaines et plus particulièrement des cheveux comprenant un pré- ou un post- traitement avec une composition comprenant au moins une silicone aminée particulière.

Les nuances naturelles des cheveux foncés peuvent être éclaircies de façon durable au moyen de traitements de décoloration. Il en va de même pour les nuances artificiellement communiquées aux cheveux par le biais des teintures directes ou d'oxydation et que l'on désire effacer.
Les compositions de décoloration utilisées dans ces traitements sont constituées de compositions aqueuses épaissies de peroxyde d'hydrogène, prêtes à l'emploi. Mais elles se présentent principalement sous forme de produits anhydres ( poudres ou crèmes ) contenant des composés alcalins (amines et silicates alcalins), et un réactif oxydant peroxygéné tels que les persulfates, perborates ou percarbonates, d'ammonium ou de métaux alcalins, que l'on dilue au moment de l'emploi avec une composition aqueuse de peroxyde d'hydrogène.
Ces traitements oxydants très efficaces induisent cependant une sensibilisation des cheveux ; les cheveux deviennent plus secs, plus difficiles à démêler, avec un toucher rêche.
D'autres traitements de décoloration utilisent des compositions prêtes à l'emploi constituées de produits anhydres (poudres ou crèmes) comprenant des agents réducteurs qui sont mélangés au moment de l'emploi avec une composition aqueuse contenant éventuellement un agent de pH. Ces traitements réducteurs peuvent également induire une sensibilisation des cheveux.

Le document EP890335 décrit une composition cosmétique pour la coloration d'oxydation des cheveux comprenant une silicone aminée qui peut être substituée par un radical alcoxy.

Jusqu'ici, pour améliorer l'état de la fibre capillaire après un traitement de décoloration, on a eu recours à des soins rincés ou non rincés, tels que des après-shampooings, des masques traitants, des crèmes traitantes, ou des sérums.
Ces soins ont l'inconvénient d'être temporaires et doivent être renouvelés après chaque lavage des cheveux.
En outre, la plupart du temps, ils alourdissent les cheveux, les rendant mous, avec un toucher enrobé glissant peu naturel.

Il existe donc un besoin d'améliorer l'état que présente les cheveux après un traitement de décoloration.

Après d'importantes études sur la question, la demanderesse a découvert de manière tout à fait inattendue et surprenante que l'utilisation, en pré- ou post- traitement sur des fibres kératiniques humaines et plus particulièrement des cheveux, d'une composition comprenant au moins une silicone aminée particulière, permettait de résoudre ce problème.
Cette découverte est à l'origine de la présente invention.

Un premier objet de l'invention concerne donc l'utilisation, en pré- ou post- traitement d'une décoloration des fibres kératiniques humaines et plus particulièrement des cheveux, d'une composition comprenant au moins une silicone aminée de formules (I) ou (II).

Ladite utilisation a notamment pour objet d'améliorer l'état des cheveux après décoloration, les cheveux sont plus doux, lisses de la racine à la pointe, individualisés, légers, souples et soyeux. Par ailleurs, ils offrent un toucher bien plus naturel qu'avec un agent conditionneur de l'art antérieur utilisé après une décoloration. En outre, ils se démêlent bien et se coiffent bien plus facilement. Le traitement selon l'invention a pour autre avantage de ne pas modifier le pouvoir éclaircissant du traitement de décoloration et les effets cosmétiques ainsi produits sont durables et visibles pendant au moins six semaines.

Par amélioration de l'état de la fibre on entend une diminution de la porosité ou de la solubilité alcaline de la fibre et une amélioration des propriétés cosmétiques et en particulier du lissage, de la douceur et de la facilité de démêlage et de coiffage.
Cet effet est rémanent, c'est à dire durable.
La porosité se mesure par la fixation à 37°C et à pH10, en 2 minutes, de la 2-nitro paraphénylènediamine à 0,25% dans un mélange éthanol / tampon pH 10 (rapport volumique 10/90).
La solubilité alcaline correspond à la perte de masse d'un échantillon de 100 mg de fibres kératiniques sous l'action de la soude décinormale pendant 30 minutes à 65°C.

Un second objet de l'invention concerne un procédé de décoloration consistant à appliquer sur les fibres kératiniques humaines et plus particulièrement les cheveux, humides ou secs, lavés ou non lavés, une composition comprenant au moins une silicone aminée de formules (I) ou (II), à la laisser agir à température ambiante ou avec apport de chaleur, à rincer ou ne pas rincer les fibres, puis à appliquer la composition décolorante, à laver les fibres, les rincer puis les sécher.

Un autre objet de l'invention concerne un procédé de décoloration consistant à appliquer sur les fibres kératiniques humaines et plus particulièrement les cheveux, une composition décolorante, à les laver au shampooing, à les rincer à l'eau, puis à appliquer sur les fibres humides ou sèches, une composition comprenant au moins une silicone aminée de formules (I) ou (II), à la laisser agir à température ambiante ou avec apport de chaleur, à rincer ou ne pas rincer les fibres, puis à les sécher. Selon ce procédé de post-traitement, la composition peut être appliquée immédiatement après décoloration ou de manière différée.

Silicones aminées

Les silicones aminées de formules (I) ou (II) selon l'invention sont les suivantes :

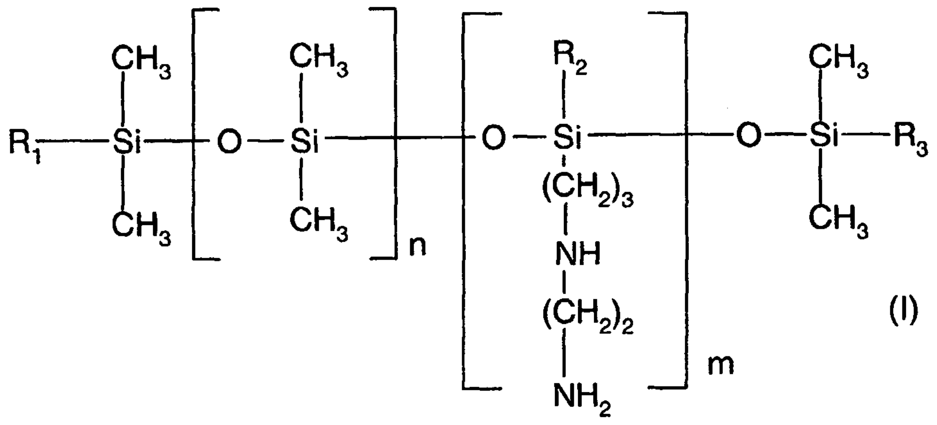

(I)

dans laquelle :

m et n sont des nombres tels que la somme (n + m) peut varier notamment de 1 à 1 000 et en particulier de 50 à 250 et plus particulièrement de 100 à 200,
n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5;
$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.

De préférence le radical alcoxy est un radical méthoxy.
Le rapport molaire Hydroxy/Alcoxy est de préférence compris entre 0,2:1 et 0,4:1 et de préférence entre 0,25:1 et 0,35: 1 et plus particulièrement est égal à 0,3.
La silicone aminée de formule (I) présente une masse moléculaire moyenne en poids allant de préférence de 2000 à 1000000 et encore plus particulièrement de 3500 à 200000.

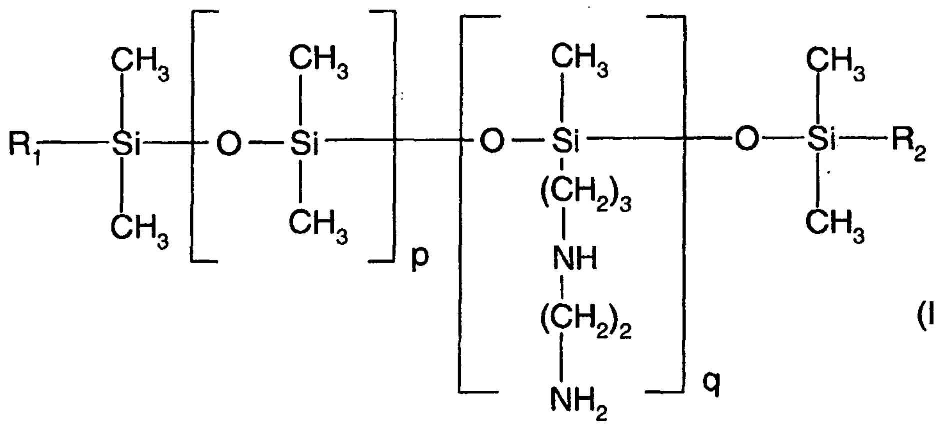

(II)

dans laquelle :

p et q sont des nombres tels que la somme (p + q) peut varier notamment de 1 à 1 000 et en particulier de 50 à 350, et plus particulièrement de 150 à 250

p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;

$R_1$, $R_2$, différents, représentent un radical hydroxy ou alcoxy en $C_1$-$C_4$, l'un au moins des radicaux $R_1$ à $R_2$ désignant un radical alcoxy.

De préférence le radical alcoxy est un radical méthoxy.

Le rapport molaire Hydroxy/Alcoxy est de préférence compris entre 1:0,8 et 1:1,1 et de préférence entre 1:0,9 et 1:1 et plus particulièrement est égal à 1:0,95.

La silicone aminée de formule (II) présente une masse moléculaire moyenne en poids allant de préférence de 2000 à 200.000, et encore plus particulièrement de 5.000 à 100.000 et plus particulièrement encore de 10.000 à 50.000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.

Les produits commerciaux correspondant à ces silicones de structure (I) ou (II) peuvent inclure dans leur composition une ou plusieurs autres silicones aminées dont la structure est différente des structures (I) et (II).

Un produit contenant des silicones aminées de structure (I) est proposé par la société WACKER sous la dénomination BELSIL ADM 652®.

Un produit contenant des silicones aminées de structure (II) est proposé par la société WACKER sous la dénomination Fluid WR 1300®.

Lorsque ces silicones aminées sont mises en oeuvre, une forme de réalisation particulièrement intéressante est leur utilisation sous forme d'émulsion huile-dans-eau. L'émulsion huile-dans-eau peut comprendre un ou plusieurs tensioactifs. Les tensioactifs peuvent être de toute nature mais de préférence cationique et/ou non ionique.

Les particules de silicone dans l'émulsion ont une taille moyenne allant généralement de 3 nm à 500 nanomètres. De préférence, notamment pour les silicones aminées de formule (II), on utilise des microémulsions de taille allant de 5 nm à 60 nanomètres et plus particulièrement de 10 nm à 50 nanomètres.

On peut utiliser selon l'invention les microémulsions de silicones aminées de formule (II) proposées sous la dénomination FINISH CT 96 E® ou SLM 28020® par la société WACKER.

De préférence la silicone aminée de formule (I) ou (II) est choisie de telle façon que l'angle de contact avec l'eau d'un cheveu traité avec une composition contenant 2% MA (matières actives) de ladite silicone selon l'invention soit compris entre 90 et 180° et de préférence entre 90 et 130°, bornes incluses.

De préférence la composition contenant la ou les silicones aminées de formule (I) ou (II) est telle que l'angle de contact d'un cheveu traité avec la dite composition est compris entre 90 et 180° et de préférence entre 90 et 130°, bornes incluses.

La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact θ entre l'eau et la surface du cheveu. Le cheveu est dit hydrophile lorsque l'angle θ est compris entre 0 et 90°, hydrophobe lorsque cet angle est compris entre 90° et 180°, bornes incluses.

Le test s'effectue avec des mèches de cheveux naturels ayant été décolorés dans les mêmes conditions puis lavés.

Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée pendant 30 secondes. La mèche essorée est laissée à l'air libre jusqu'à ce qu'elle soit complètement sèche.

Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.

Parallèlement, le périmètre du cheveu (P) est mesuré *via* une observation microscopique. La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, Γlv la tension interfaciale

liquide / vapeur de l'eau en J/m$^2$ et $\theta$ l'angle de contact.
Le produit SLM 28020® de WACKER à 12% dans l'eau (soit 2% en matières actives) conduit à un angle de contact de 93° selon le test indiqué ci-dessus.

La silicone aminée est utilisée de préférence dans la composition de pré- ou de post-traitement en une quantité allant de 0,01 à 20% en poids du poids total de la composition. Plus préférentiellement, cette quantité va de 0,1 à 15% en poids et encore plus particulièrement de 0,5 à 10 % en poids.

La composition de pré- ou de post- traitement peut contenir tous les ingrédients classiquement utilisés en cosmétique et en particulier dans le domaine capillaire. En particulier elle peut contenir des tensioactifs et/ou des polymères additionnels. Ces tensioactifs et ces polymères peuvent être de nature non-ionique, cationique, anionique ou amphotère.
Parmi les polymères additionnels, les silicones aminées autres que celles de l'invention sont particulièrement préférées.

La composition de pré- ou de post- traitement présente un pH allant de 2 à 11 et de préférence de 4 à 9 quand elle est aqueuse.
Elle peut se présenter sous diverses formes telles que lotions, gels, crèmes, shampooings, sticks, mousses, sprays. Pour certaines de ces formes, elle peut être conditionnée en flacon pompe ou dans un récipient aérosol. Dans le cas de l'aérosol, la composition est associée à un agent propulseur qui peut être par exemple un alcane ou un mélange d'alcane, du diméthyl éther, de l'azote, du protoxyde d'azote, du gaz carbonique ou des halgénoalcanes, ainsi que leurs mélanges.
Une forme particulièrement préférée selon l'invention est la forme shampooing.
Dans ce cas, la composition contient au moins un agent tensioactif qui est de préférence anionique. De préférence alors, elle contient un mélange de tensioactifs dont au moins un agent tensioactif anionique, le ou les autres tensioactifs étant préférentiellement non ioniques ou amphotères.

Comme il est indiqué ci dessus, la composition de post- traitement peut être appliquée immédiatement après décoloration ou de manière différée. Par différée, on entend une application se faisant quelques heures, un jour ou plusieurs jours (de 1 à 60 jours) après la décoloration.
De préférence, plusieurs applications sont effectuées entre deux décolorations.
Le nombre d'applications entre deux décolorations est de préférence compris entre 1 et 60 et encore plus préférentiellement entre 2 et 30.

La composition de pré- ou de post- traitement peut être utilisée en mode rincé ou en mode non-rincé, c'est à dire que son application est suivie ou non d'un rinçage.

Dans le premier cas, le temps de pose de la composition de pré- ou de post- traitement est compris entre quelques secondes et 60 minutes et de préférence entre 30 secondes et 15 minutes.

La température d'application de la composition de pré- ou de post- traitement peut varier de 10°C à 70°C. De préférence l'application s'effectuera entre 20 et 60°C et plus particulièrement à la température ambiante.

Les exemples suivants sont destinés à illustrer l'invention. Celle-ci n'est cependant pas limitée à ces modes de réalisation.

EXEMPLES

On a préparé les trois compositions selon l'invention suivantes :
(exprimées en grammes de matière active)

| **Composition A** | |
|---|---|
| Polydiméthylsiloxane de formule (I) selon l'invention proposé sous la dénomination BELSIL ADM 652® par la société WACKER | 2 |
| Cyclopentadimethylsiloxane q.s.p | 100 |

| **Composition B** | |
|---|---|
| Polydiméthylsiloxane de formule (II) selon l'invention proposé sous la dénomination SLM 28020® par la société WACKER | 2 |
| Eau déminéralisée q.s.p | 100 |

| Composition C | |
|---|---|
| Alcool cétylstéarylique / laurylsulfate de sodium / myristate de cétyle / alcool myristique (62/20/8/10) | 12 |
| Alcool oléique oxyéthyléné (20 OE) | 0.1 |
| Glycérine | 0.5 |
| Polydiméthylsiloxane de formule (I) selon l'invention proposé sous la dénomination BELSIL ADM 652 par la société WACKER | 2 |
| Eau déminéralisée q.s.p | 100 |

Les compositions A, B et C ont été appliquées chacune sur des cheveux naturels châtain :

-1/ en pré-traitement d'une décoloration oxydante utilisant le produit du commerce PLATIFIZ® de la société L'OREAL ;

-2/ en post-traitement d'une décoloration oxydante utilisant le produit du commerce PLATIFIZ® de la société L'OREAL.

En pré-traitement, les cheveux ont ainsi été traités avec ces compositions A, B et C pendant 30 minutes à la température de 37°C, rincés à l'eau, décolorés au PLATIFIZ® et rincés de nouveau abondamment avant séchage.
En post-traitement, les cheveux ont subi une décoloration PLATIFIZ®, ont été rincés à l'eau avant d'être traités par les compositions A, B et C puis rincés de nouveau abondamment avant d'être séchés.
A l'issue de ces traitements, les cheveux ont été plus lisses, plus doux, plus faciles à démêler qu'en l'absence de pré- ou de post-traitement.
En outre, le niveau de décoloration n'a pas été altéré par lesdits traitements selon l'invention.

## Revendications

1. Utilisation, en pré- ou en post- traitement d'une décoloration des fibres kératiniques humaines d'une composition comprenant au moins une silicone aminée de formules (I) ou (II) suivantes :

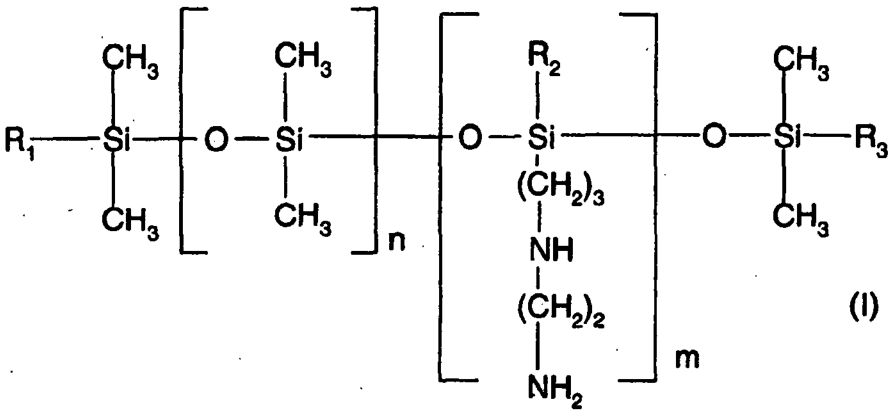

(I)

formule (I) dans laquelle :
m et n sont des nombres tels que la somme (n + m) varie de 1 à 1 000,
n désignant un nombre de 0 à 999
et m désignant un nombre de 1 à 1 000;
$R_1$, $R_2$, $R_3$, identiques ou différents, représentent un radical hydroxy ou alcoxy en $C_1$-$C_4$ ; l'un au moins des radicaux $R_1$ à $R_3$ désignant un radical alcoxy ;

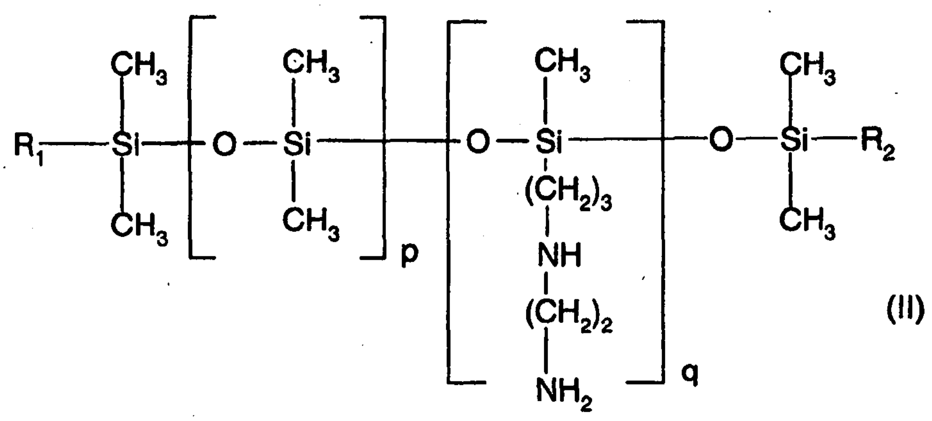

(II)

formule (II) dans laquelle :
p et q sont des nombres tels que la somme (p + q) varie de 1 à 1 000,
p désignant un nombre de 0 à 999
et q désignant un nombre de 1 à 1 000;
$R_1$, $R_2$, différents, représentent un radical hydroxy ou alcoxy en $C_1$-$C_4$, l'un au moins des radicaux $R_1$ à $R_2$ désignant un radical alcoxy.

2. Utilisation selon la revendication 1, **caractérisée par le fait que** le radical alcoxy $C_1$-$C_4$ désigne le radical méthoxy.

3. Utilisation selon l'une quelconque des revendications précédentes, **caractérisée par le fait que** pour les silicones aminées de formule (I), le rapport molaire Hydroxy/Alcoxy est compris entre 0,2:1 et 0,4:1.

4. Utilisation selon l'une quelconque des revendications 1 à 2, **caractérisée par le fait que** pour les silicones aminées de formule (II), le rapport molaire Hydroxy/Alcoxy est compris entre 1:0,8 et 1:1,1.

5. Utilisation selon l'une quelconque des revendications 1 à 3, **caractérisée par le fait que** la silicone aminée de formule (I) présente une masse moléculaire moyenne en poids allant de 2000 à 1000000.

6. Utilisation selon l'une quelconque des revendications 1 à 2 et 4, **caractérisée par le fait que** la silicone aminée de formule (II) présente une masse moléculaire moyenne en poids allant de 2000 à 200.000.

7. Utilisation selon l'une quelconque des revendications précédentes, **caractérisée par le fait que** la silicone aminée de formule (I) ou (II) est sous forme d'émulsion huile-dans-eau comprenant des agents tensioactifs.

8. Utilisation selon la revendication 7, **caractérisée par le fait que** l'émulsion comprend au moins un agent tensioactif cationique et/ou non ionique.

9. Utilisation selon l'une quelconque des revendications 7 ou 8, **caractérisée par le fait que** les particules de silicone dans l'émulsion ont une taille allant de 3 nm à 500 nanomètres.

10. Utilisation selon l'une quelconque des revendications précédentes, **caractérisée par le fait que** la silicone aminée de formules (I) ou (II) est présente dans la composition de pré- ou de post- traitement en une quantité allant de 0,01 à 20% en poids du poids total de la composition.

11. Utilisation selon la revendication 10, **caractérisée par le fait qu'**elle est présente en une quantité allant de 0,1 à 15% en poids en poids du poids total de la composition.

12. Utilisation selon la revendication 11, **caractérisée par le fait qu'**elle est présente en une quantité allant de 0,5 à 10 % en poids du poids total de la composition.

13. Utilisation selon l'une quelconque des revendications précédentes, **caractérisée par le fait que** la composition de

pré- ou de post- traitement se présente sous forme de lotions, gels, crèmes, shampooings, sticks, mousses, sprays.

**14.** Utilisation selon l'une quelconque des revendications précédentes, **caractérisée par le fait que** la composition de pré- ou de post- traitement est conditionnée en flacon pompe ou dans un récipient aérosol.

**15.** Utilisation selon la revendication 14, **caractérisée par le fait que** la composition de pré- ou de post- traitement est associée à au moins un agent propulseur choisi parmi les alcanes, le diméthyl éther, l'azote, le protoxyde d'azote, le gaz carbonique ou les halogénoalcanes.

**16.** Utilisation selon l'une quelconque des revendications précédentes, **caractérisée par le fait que** la composition de pré- ou de post- traitement comprend au moins un agent tensioactif de nature non-ionique, cationique, anionique ou amphotère.

**17.** Utilisation selon la revendication 16, **caractérisée par le fait que** la composition de pré- ou de post- traitement comprend un mélange d'agents tensioactifs comprenant au moins un agent tensioactif anionique, le ou les autres agents tensioactifs étant non ioniques ou amphotères.

**18.** Utilisation selon l'une quelconque des revendications précédentes, **caractérisée par le fait que** la composition de pré- ou de post- traitement comprend au moins un polymère additionnel autre que la silicone aminée de formules (I) ou (II).

**19.** Utilisation selon la revendication 18, **caractérisée par le fait que** le polymère est de nature non-ionique, cationique, anionique ou amphotère.

**20.** Utilisation selon la revendication 19, **caractérisée par le fait que** le polymère est une silicone aminée différente de la silicone aminée de formules (I) ou (II).

**21.** Utilisation selon l'une quelconque des revendications précédentes, **caractérisée par le fait que** la composition de pré- ou de post- traitement présente un pH allant de 2 à 11.

**22.** Utilisation selon la revendication 1 pour améliorer l'état des fibres kératiniques humaines après une décoloration.

**23.** Procédé de décoloration des fibres kératiniques humaines **caractérisé par le fait qu'**il consiste à appliquer sur les fibres, dans une première étape, une composition de pré- traitement comprenant au moins une silicone aminée de formules (I) ou (II) et telle que décrite à l'une quelconque des revendications 1 à 12, puis, après avoir rincé ou non rincé les fibres et les avoir séchées ou non séchées, dans une seconde étape, appliquer une composition de décoloration, à laver les fibres, les rincer puis les sécher.

**24.** Procédé de décoloration des fibres kératiniques humaines **caractérisé par le fait qu'**il consiste à appliquer sur les fibres, dans une première étape, une composition décolorante, à la laisser agir pendant un temps suffisant pour décolorer, puis, après avoir rincé ou non rincé les fibres et les avoir séchées ou non séchées, dans une seconde étape, appliquer une composition de post-traitement comprenant au moins une silicone aminée de formules (I) ou (II), et telle que décrite à l'une quelconque des revendications 1 à 12, ladite composition de post-traitement étant appliquée, soit immédiatement, soit en différé, et les applications de ladite composition pouvant être répétées entre deux décolorations.

**25.** Procédé selon l'une quelconque des revendications 23 ou 24, **caractérisé par le fait que** la composition de pré- ou de post- traitement est posée pendant un temps allant de quelques secondes à 60 minutes.

## Claims

**1.** Use, as a pre- or post-treatment of a process for bleaching human keratin fibres, of a composition comprising at least one aminosilicone of formula (I) or (II) below:

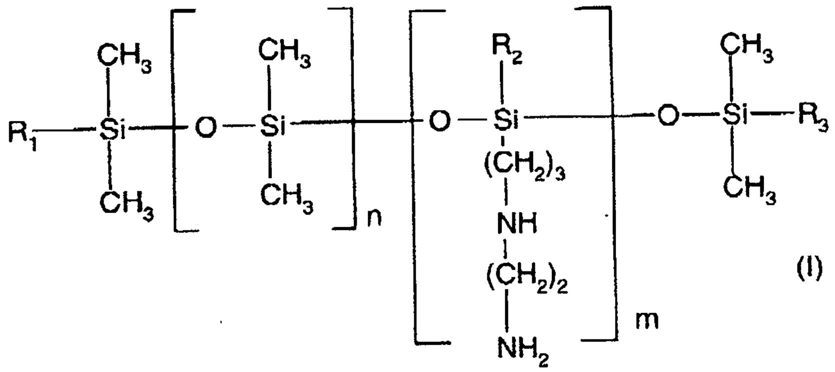

(I)

in which formula (I):
m and n are numbers such that the sum (n + m) ranges from 1 to 1000,
n denoting a number from 0 to 999 and m denoting a number from 1 to 1000;
$R_1$, $R_2$ and $R_3$, which may be identical or different, represent a hydroxyl or C1-C4 alkoxy radical, at least one of the radicals $R_1$ to $R_3$ denoting an alkoxy radical;

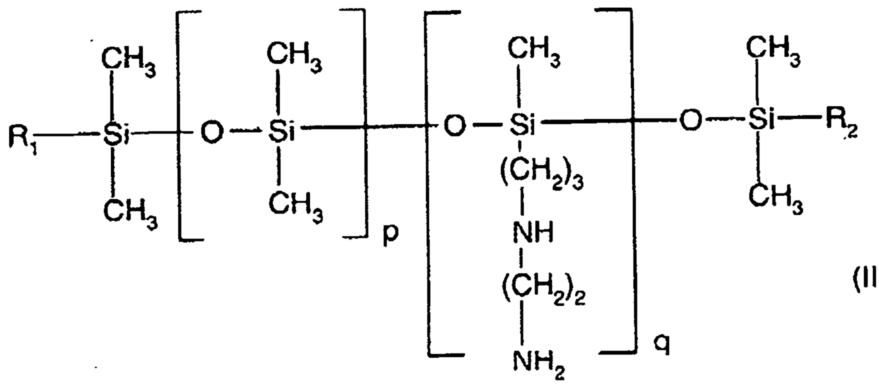

(II)

in which formula (II):
p and q are numbers such that the sum (p + q) ranges from 1 to 1000,
p denoting a number from 0 to 999 and q denoting a number from 1 to 1000;
$R_1$ and $R_2$, which are different, represent a hydroxyl or C1-C4 alkoxy radical, at least one of the radicals $R_1$ and $R_2$ denoting an alkoxy radical.

2. Use according to Claim 1, **characterized in that** the C1-C4 alkoxy radical denotes a methoxy radical.

3. Use according to either of the preceding claims, **characterized in that**, for the aminosilicones of formula (I), the hydroxyl/alkoxy molar ratio is between 0.2:1 and 0.4:1.

4. Use according to either of Claims 1 and 2, **characterized in that**, for the aminosilicones of formula (II), the hydroxyl/alkoxy molar ratio is between 1:0.8 and 1:1.1.

5. Use according to any one of Claims 1 to 3, **characterized in that** the aminosilicone of formula (I) has a weight-average molecular mass ranging from 2000 to 1 000 000.

6. Use according to any one of Claims 1, 2 and 4, **characterized in that** the aminosilicone of formula (II) has a weight-average molecular mass ranging from 2000 to 200 000.

7. Use according to any one of the preceding claims, **characterized in that** the aminosilicone of formula (I) or (II) is in the form of an oil-in-water emulsion comprising surfactants.

**8.** Use according to Claim 7, **characterized in that** the emulsion comprises at least one cationic and/or nonionic surfactant.

**9.** Use according to either of Claims 7 and 8, **characterized in that** the silicone particles in the emulsion range in size from 3 nm to 500 nanometres.

**10.** Use according to any one of the preceding claims, **characterized in that** the aminosilicone of formula (I) or (II) is present in the pre- or post-treatment composition in an amount ranging from 0.01% to 20% by weight relative to the total weight of the composition.

**11.** Use according to Claim 10, **characterized in that** aminosilicone is present in an amount ranging from 0.1% to 15% by weight relative to the total weight of the composition.

**12.** Use according to Claim 11, **characterized in that** aminosilicone is present in an amount ranging from 0.5% to 10% by weight relative to the total weight of the composition.

**13.** Use according to any one of the preceding claims, **characterized in that** the pre- or post-treatment composition is in the form of lotions, gels, creams, shampoos, sticks, mousses or sprays.

**14.** Use according to any one of the preceding claims, **characterized in that** the pre- or post-treatment composition is packaged in a pump-dispenser bottle or in an aerosol container.

**15.** Use according to Claim 14, **characterized in that** the pre- or post-treatment composition is combined with at least one propellant chosen from alkanes, dimethyl ether, nitrogen, nitrous oxide, carbon dioxide and haloalkanes.

**16.** Use according to any one of the preceding claims, **characterized in that** the pre- or post-treatment composition comprises at least one surfactant of nonionic, cationic, anionic or amphoteric nature.

**17.** Use according to Claim 16, **characterized in that** the pre- or post-treatment composition comprises a mixture of surfactants comprising at least one anionic surfactant, the other surfactant(s) being nonionic or amphoteric.

**18.** Use according to any one of the preceding claims, **characterized in that** the pre- or post-treatment composition comprises at least one additional polymer other than the aminosilicone of formula (I) or (II).

**19.** Use according to Claim 18, **characterized in that** the polymer is of nonionic, cationic, anionic or amphoteric nature.

**20.** Use according to Claim 19, **characterized in that** the polymer is an aminosilicone different from the aminosilicone of formula (I) or (II).

**21.** Use according to any one of the preceding claims, **characterized in that** the pre- or post-treatment composition has a pH ranging from 2 to 11.

**22.** Use according to Claim 1, for improving the condition of human keratin fibres after bleaching.

**23.** Process for bleaching human keratin fibres, **characterized in that** it consists in applying to the fibres, in a first step, a pretreatment composition comprising at least one aminosilicone of formula (I) or (II) as described in any one of Claims 1 to 12, and then, after having optionally rinsed the fibres, and after having optionally dried them, in a second step, in applying a bleaching composition, washing the fibres, rinsing them and then drying them.

**24.** Process for bleaching human keratin fibres, **characterized in that** it consists in applying to the fibres, in a first step, a bleaching composition, in leaving it to act for a time that is sufficient to bleach, and then, after optionally rinsing the fibres and optionally drying them, in a second step, in applying a post-treatment composition comprising at least one aminosilicone of formula (I) or (II) as described in any one of Claims 1 to 12, the said post-treatment composition being applied either immediately or after an interval, and the applications of the said composition possibly being repeated between two bleaching operations.

**25.** Process according to either of Claims 23 and 24, **characterized in that** the pre- or post-treatment composition is left to act for a time ranging from a few seconds to 60 minutes.

## Patentansprüche

1. Verwendung einer Zusammensetzung, die mindestens ein aminiertes Silicon der folgenden Formeln (I) oder (II) enthält, für die Vorbehandlung oder Nachbehandlung bei einer Entfärbung menschlicher Keratinfasern:

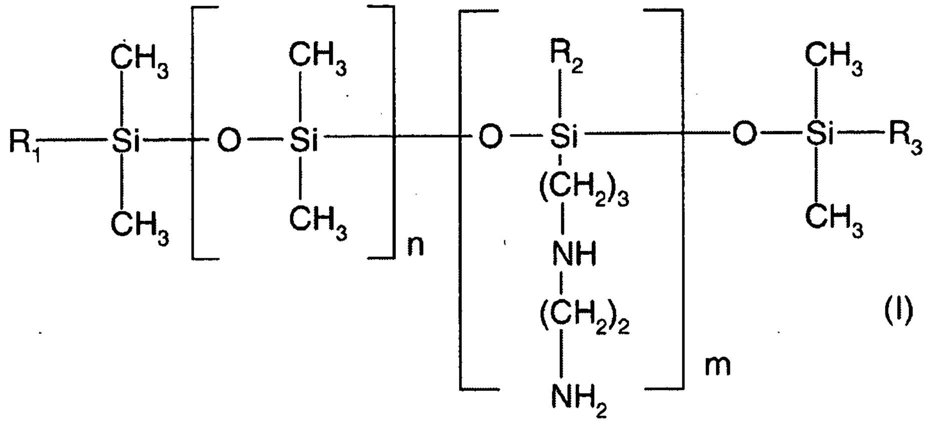

(I)

in der Formel (I):
m und n bedeuten Zahlen, die so gewählt sind, dass die Summe (n + m) im Bereich von 1 bis 1000 liegt,
n ist eine Zahl im Bereich von 0 bis 999 und m ist eine Zahl im Bereich von 1 bis 1000,
$R_1$, $R_2$ und $R_3$, die gleich oder verschieden sind, bedeuten eine Hydroxygruppe oder eine $C_{1-4}$-Alkoxygruppe, wobei mindestens eine der Gruppen $R_1$ bis $R_3$ eine Alkoxygruppe bedeutet;

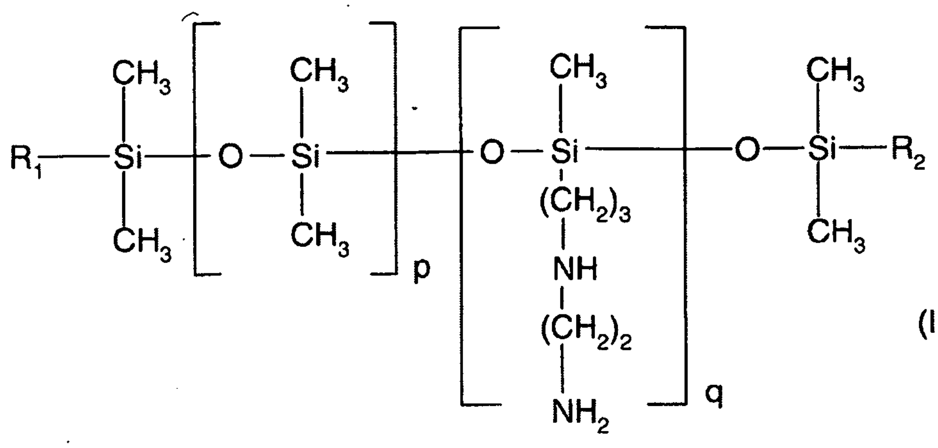

(II)

in der Formel (II):
p und q bedeuten Zahlen, die so gewählt sind, dass die Summe (p + q) im Bereich von 1 bis 1000 liegt,
p ist eine Zahl im Bereich von 0 bis 999 und q ist eine Zahl im Bereich von 1 bis 1000,
$R_1$ und $R_2$, die verschieden sind, bedeuten eine Hydroxygruppe oder eine $C_{1-4}$-Alkoxygruppe, wobei mindestens eine der Gruppen $R_1$ bis $R_2$ eine Alkoxygruppe bedeutet.

2. Verwendung nach Anspruch 1, **dadurch gekennzeichnet, dass** die $C_{1-4}$-Alkoxygruppe die Methoxygruppe ist.

3. Verwendung nach einem der vorhergehenden Ansprüche, **dadurch gekennzeichnet, dass** für die aminierten Silicone der Formel (I) das Molverhältnis Hydroxy/Alkoxy im Bereich von 0,2:1 bis 0,4:1 liegt.

4. Verwendung nach einem der Ansprüche 1 bis 2, **dadurch gekennzeichnet, dass** für die aminierten Silicone der Formel (II) das Molverhältnis Hydroxy/Alkoxy im Bereich von 1:0,8 bis 1:1,1 liegt.

5. Verwendung nach einem der Ansprüche 1 bis 3, **dadurch gekennzeichnet, dass** das aminierte Silicon der Formel (I) eine gewichtmittlere Molmasse von 2.000 bis 1.000.000 aufweist.

6. Verwendung nach einem der Ansprüche 1 bis 2 und 4, **dadurch gekennzeichnet, dass** das aminierte Silicon der Formel (II) eine gewichtmittlere Molmasse von 2.000 bis 200.000 aufweist.

7. Verwendung nach einem der vorhergehenden Ansprüche, **dadurch gekennzeichnet, dass** das aminierte Silicon der Formel (I) oder (II) in Form einer Öl-in-Wasser-Emulsion vorliegt, die grenzflächenaktive Stoffe enthält.

8. Verwendung nach Anspruch 7, **dadurch gekennzeichnet, dass** die Emulsion mindestens einen kationischen und/ oder nichtionischen grenzflächenaktiven Stoff enthält.

9. Verwendung nach einem der Ansprüche 7 oder 8, **dadurch gekennzeichnet, dass** die Siliconpartikel in der Emulsion eine Größe im Bereich von 3 bis 500 nm aufweisen.

10. Verwendung nach einem der vorhergehenden Ansprüche, **dadurch gekennzeichnet, dass** das aminierte Silicon der Formel (I) oder (II) in der Zusammensetzung für die Vor- oder Nachbehandlung in einem Mengenanteil von 0,01 bis 20 Gew.-% des Gesamtgewichts der Zusammensetzung enthalten ist.

11. Verwendung nach Anspruch 10, **dadurch gekennzeichnet, dass** es in einem Mengenanteil von 0,1 bis 15 Gew.-% des Gesamtgewichts der Zusammensetzung enthalten ist.

12. Verwendung nach Anspruch 11, **dadurch gekennzeichnet, dass** es in einem Mengenanteil von 0,5 bis 10 Gew.-% des Gesamtgewichts der Zusammensetzung enthalten ist.

13. Verwendung nach einem der vorhergehenden Ansprüche, **dadurch gekennzeichnet, dass** die Zusammensetzung für die Vor- oder Nachbehandlung als Lotion, Gel, Creme, Haarwaschmittel, Stift, Schaum oder Spray vorliegt.

14. Verwendung nach einem der vorhergehenden Ansprüche, **dadurch gekennzeichnet, dass** die Zusammensetzung für die Vor- oder Nachbehandlung in einem Pumpflakon oder einem Aerosolbehälter konfektioniert ist.

15. Verwendung nach Anspruch 14, **dadurch gekennzeichnet, dass** die Zusammensetzung für die Vor- oder Nachbehandlung mindestens ein Treibmittel enthält, das unter den Alkanen, Dimethylether, Stickstoff, Distickstoffoxid, Kohlendioxid oder Halogenalkanen ausgewählt ist.

16. Verwendung nach einem der vorhergehenden Ansprüche, **dadurch gekennzeichnet, dass** die Zusammensetzung für die Vor- oder Nachbehandlung mindestens einen grenzflächenaktiven Stoff vom nichtionischen, kationischen, anionischen oder amphoteren Typ enthält.

17. Verwendung nach Anspruch 16, **dadurch gekennzeichnet, dass** die Zusammensetzung für die Vor- oder Nachbehandlung ein Gemisch von grenzflächenaktiven Stoffen enthält, das mindestens einen anionischen grenzflächenaktiven Stoff umfasst, wobei der oder die anderen grenzflächenaktiven Stoffe nichtionisch oder amphoter sind.

18. Verwendung nach einem der vorhergehenden Ansprüche, **dadurch gekennzeichnet, dass** die Zusammensetzung für die Vor- oder Nachbehandlung mindestens ein ergänzendes Polymer enthält, das von den aminierten Siliconen der Formel (I) oder (II) verschieden ist.

19. Verwendung nach Anspruch 18, **dadurch gekennzeichnet, dass** das Polymer vom nichtionischen, kationischen, anionischen oder amphoteren Typ ist.

20. Verwendung nach Anspruch 19, **dadurch gekennzeichnet, dass** das Polymer ein aminiertes Silicon ist, das von den Siliconen der Formel (I) oder (II) verschieden ist.

21. Verwendung nach einem der vorhergehenden Ansprüche, **dadurch gekennzeichnet, dass** die Zusammensetzung für die Vor- oder Nachbehandlung einen pH-Wert im Bereich von 2 bis 11 aufweist.

22. Verwendung nach Anspruch 1, um den Zustand menschlicher Keratinfasern nach einer Entfärbung zu verbessern.

**23.** Verfahren zum Entfärben menschlicher Keratinfasern, **dadurch gekennzeichnet, dass** es darin besteht, in einem ersten Schritt eine Zusammensetzung für die Vorbehandlung, die mindestens ein aminiertes Silicon der Formel (I) oder (II) enthält und wie sie in einem der Ansprüche 1 bis 12 beschrieben ist, auf die Fasern aufzutragen, und dann, nachdem die Fasern gegebenenfalls gespült und gegebenenfalls getrocknet wurden, in einem zweiten Schritt eine Zusammensetzung zum Entfärben aufzutragen, worauf die Fasern gewaschen, gespült und dann getrocknet werden.

**24.** Verfahren zum Entfärben menschlicher Keratinfasern, **dadurch gekennzeichnet, dass** es darin besteht, in einem ersten Schritt eine Zusammensetzung zum Entfärben auf die Fasern aufzutragen und während einer Zeitspanne einwirken zu lassen, die zum Entfärben ausreichend ist, und dann, nachdem die Fasern gegebenenfalls gespült und gegebenenfalls getrocknet wurden, in einem zweiten Schritt eine Zusammensetzung für die Nachbehandlung, die mindestens ein aminiertes Silicon der Formel (I) oder (II) enthält und wie sie in einem der Ansprüche 1 bis 12 beschrieben ist, aufzutragen, wobei die Zusammensetzung für die Nachbehandlung entweder sofort oder später aufgetragen wird und die Anwendungen dieser Zusammensetzung zwischen zwei Entfärbungen wiederholt werden können.

**25.** Verfahren nach einem der Ansprüche 23 oder 24, **dadurch gekennzeichnet, dass** Zusammensetzung für die Vor- oder Nachbehandlung während einer Zeitspanne von einigen Sekunden bis 60 min einwirken gelassen wird.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 890335 A